# EUROPÄISCHE PATENTSCHRIFT

(11) **EP 0 983 219 B1**
(45) Veröffentlichungstag und Bekanntmachung des Hinweises auf die Patenterteilung: **06.11.2002**
(21) Anmeldenummer: 98925581.5
(22) Anmeldetag: 12.05.1998
(51) Int. Cl.: C07C 29/136, B01J 23/42, B01J 23/68

(54) **VERFAHREN ZUR HERSTELLUNG VON ALIPHATISCHEN ALKOHOLEN**
METHOD FOR PRODUCING ALIPHATIC ALCOHOLS
PROCEDE DE PRODUCTION D'ALCOOLS ALIPHATIQUES

(30) Priorität: 16.05.1997 DE 19720657
(43) Veröffentlichungstag der Anmeldung: 08.03.2000
(73) Patentinhaber: BASF AKTIENGESELLSCHAFT, 67056 Ludwigshafen (DE)
(72) Erfinder: FISCHER, Rolf, D-69121 Heidelberg (DE); PINKOS, Rolf, D-67098 Bad Dürkheim (DE); WULFF-DÖRING, Joachim, D-67227 Frankenthal (DE)
(74) Vertreter: Isenbruck, Günter, Dr.
(86) Internationale Anmeldenummer: EP9802777
(87) Internationale Veröffentlichungsnummer: WO98052891

(56) Entgegenhaltungen:
- EP-A- 0 417 867
- GB-A- 1 551 741
- US-A- 4 214 106

## Beschreibung

Die Erfindung betrifft ein Verfahren zur Herstellung von aliphatischen Diolen oder Lactonen durch Hydrierung von aliphatischen Dicarbonsäuren oder Anhydriden oder Estern davon in Gegenwart eines Katalysators.

Unterschiedliche Verfahren zur Hydrierung von aliphatischen Carbonsäuren zu aliphatischen Alkoholen sind bekannt.

In K. Yoshino et.al., "Hydrogenation of carboxylic acids by rhenium-osmium bimetallic catalyst", JAOCS, Band 67, Nr. 1, Januar 1990, Seiten 21 bis 24 ist die Hydrierung von Hexansäure und Decansäure zu den entsprechenden Alkoholen in Gegenwart eines Katalysators beschrieben, der Re₂O₇ und OSO₄ auf einem Kohlenstoffträger enthält.

In der DE-A-27 15 667 ist ein Verfahren zur Herstellung von 1,4-Butandiol durch Hydrierung von Maleinsäureanhydrid, Maleinsäure oder Fumarsäure beschrieben, wobei als Katalysator Pd und Re auf einem speziellen Silikoacetat eingesetzt wird. Die Reaktionstemperatur beträgt 205 bis 230°C.

In der EP-B-0 417 867 sind Katalysatoren für die Hydrierung von Carbonsäuren und deren Anhydriden zu Alkoholen oder Estern beschrieben. Als Katalysatoren werden beispielsweise Pd, Pd/Re, Ag/Pd, Ag/Pd/Re auf Kohlenstoff eingesetzt. Es werden die Umsetzungen von Essigsäure zu Ethanol und von Maleinsäureanhydrid zu gamma-Butyrolacton beschreiben.

Die Umsetzung wird bei Temperaturen im Bereich von 194 bis 251°C durchgeführt

In GB 1,551,741 ist ein Verfahren zur Herstellung von 1,4-Butandiol durch Hydrierung von Maleinsäure bzw. Maleinsäureanhydrid beschrieben, wobei der Katalysator Platin, Rhenium und Palladium enthält.

In der US 4,214,106 ist ein Verfahren zur Herstellung von Ethylenglykol aus Glykolsäure beschrieben. Die Umsetzung wird an Pd/Re, Pd/Ag, Ru/Rh, Pd/Au, Re/Ag, PVRh oder Pd/Re/Ag als Katalysatoren bei einer Temperatur im Bereich von 145 bis 241°C durchgeführt.

Die bekannten Katalysatoren weisen nicht in allen Anwendungen genügend hohe Aktivitäten oder Selektivitäten auf.

Zudem besteht beim Einsatz von Carbonsäuren, insbesondere in Form von wäßrigen Lösungen, die Gefahr von Korrosion an den Werkstoffen der Apparaturen, die mit den Carbonsäuren in Berührung kommen, beispielsweise Reaktionsgefäßen. Aus diesem Grund müssen entweder sehr dicke Stähle oder edle und damit kostspielige Materialien für die Apparaturen verwendet werden. Es besteht somit Nachfrage nach einem Verfahren, bei dem das Problem der Korrosion deutlich vermindert ist

Aufgabe der vorliegenden Erfindung ist die Bereitstellung eines Verfahrens zur Herstellung von aliphatischen Diolen oder Lactonen durch Hydrierung von aliphatischen Dicarbonsäuren mit mindestens 3 Kohlenstoffatomen oder Anhydriden oder Estern davon in Gegenwart eines Pt und Re in metallischer oder oxidischer Form enthaltenden Katalysators, das die Nachteile der bekannten Verfahren vermeidet

Die Aufgabe wird erfindungsgemäß gelöst durch Verwendung eines Katalysators, der neben Pt und Re jeweils in metallischer und oxidischer Form ferner mindestens ein weiteres Element aus den Gruppen 5 bis 12 und 14 und der Lanthaniden des Periodensystems der Elemente, ausgenommen Palladium, in metallischer oder oxidischer Form enthält.

Die Erfindung betrifft auch die Verwendung eines derartigen Katalysators zur Hydrierung von aliphatischen Dicarbonsäuren oder Anhydriden oder Estern davon.

Erfindungsgemäß wurde gefunden, daß die vorstehend aufgeführte Umsetzung bei Verwendung des erfindungsgemäßen Katalysators bei niedrigen Temperaturen, vorzugsweise von maximal 200°C, durchgeführt werden kann, was zu einer sehr starken Verringerung des Korrosionsproblems in den Apparaturen führt.

Der erfindungsgemäß eingesetzte Katalysator enthält oder insbesondere besteht aus Pt, Re und mindestens einem weiteren Element aus den Gruppen 5 bis 12 und 14 und der Lanthaniden des Periodensystems der Elemente (IV. Hauptgruppe, I., II., V., VI., VII., VIII. Nebengruppe des Periodensystems der Elemente), ausgenommen Palladium, jeweils in metallischer oder oxidischer Form, gegebenenfalls auf einem Träger.

Der Katalysator ist herstellbar durch Reduktion einer wäßrigen Aufschlämmung und/oder Lösung von Oxiden, Oxidhydraten, Carbonaten, Nitraten, Carboxylaten, Chelaten, Sulfaten, Phosphaten und/oder Halogeniden von Pt, Re und mindestens einem weiteren Element aus den Gruppen 5 bis 12 und 14 und der Lanthaniden des Periodensystems der Elemente, ausgenommen Palladium.

Vorzugsweise stammt das mindestens eine weitere Element aus den Gruppen 6, 10 und 11 des Periodensystems der Elemente. Es wird in metallischer oder oxidischer Form eingesetzt. Besonders bevorzugt sind die Elemente Sn, V, Cr, Mo, W, Mn, Fe, Ru, Os, Co, Ni, Cu, Ag, Au, Zn, La und Ce. Insbesondere bevorzugt sind Mo, Ag und/oder Au in metallischer oder oxidischer Form. Gemäß einer Ausführungsform der Erfindung enthält der Katalysator nur ein weiteres Element in metallischer oder oxidischer Form.

Der Katalysator kann als Voll- oder Trägerkatalysator eingesetzt werden. Beim Einsatz als Trägerkatalysator können als Trägermaterial alle geeigneten Materialien eingesetzt werden, beispielsweise Aktivkohlen, SiO₂, Al₂O₃, TiO₂, ZrO₂, Tonerden, wie Montmorillonite, Zeolithe oder Gemische davon. Der Katalysator kann auf unterschiedliche Weise hergestellt werden. Beispielsweise ist der Katalysator herstellbar durch Reduktion einer wäßrigen Aufschlämmung und/oder Lösung von Oxiden, Oxidhydraten, Carbonaten, Nitraten, Carboxylaten, Chelaten, insbesondere mit 1,3-Diketoverbindungen, Sulfaten, Phosphaten und/oder Halogeniden von Pt, Re und mindestens einem weiteren Element aus den Gruppen 5 bis 12 und 14 und der Lanthaniden des Periodensystems der Elemente. Die Herstellung kann dabei so erfolgen, daß alle Komponenten zusammen vorgelegt werden und bevorzugt mit Wasserstoff reduziert werden. Die Reduktion kann jedoch auch nacheinander vor sich gehen. Die Aktivierung oder Reduktion des Katalysators beziehungsweise der Katalysatorvorläufer erfolgt dabei vorzugsweise bei Temperaturen von 200 bis 500°C, besonders bevorzugt von 210 bis 400°C, insbesondere von 220 bis 300°C. Nach der Reduktion liegen die Katalysatoren oft nicht oder nur zu einem geringen Teil als intermetallische Verbindungen vor.

Beispielsweise werden in Wasser PtO₂, eine Re-Verbindung wie Re₂O₇ und mindestens eine weitere Verbindung der dritten Komponente vorgelegt und sodann mit Wasserstoff reduziert. Der so erhaltene Katalysator kann direkt zur Hydrierung eingesetzt werden. Trägerkatalysatoren können beispielsweise so hergestellt werden, daß sich Platinoxid oder Platinoxidhydrat bereits auf dem Träger befinden, wobei das Pt/Träger-Gemisch durch Tränkung oder gemeinsame Fällung von Platinoxid- oder Platinoxidhydrat-Vorläufer und Trägermaterial und anschließende Calcinierung hergestellt werden kann. Die Re-Verbindungen sowie die weitere Komponente können durch Tränkung oder Fällung zusätzlich aufgebracht werden. Dabei kann beispielsweise das Platinoxid oder Platinoxidhydrat zuvor auf dem Träger bereits reduziert worden sein.

Das Gewichtsverhältnis von Pt zu Re beziehungsweise Pt zu dem mindestens einen weiteren Element beträgt vorzugsweise 100 bis 0,01, besonders bevorzugt 50 bis 0,05, insbesondere 10 bis 0,1.

Vorzugsweise wird Pt vor der Reduktion oder Aktivierung in Form des Oxids oder Oxidhydrats eingesetzt. Insbesondere liegt die Pt-Komponente als PtO₂ vor. Als Re-Quelle können übliche Re-Verbindungen eingesetzt werden, vorzugsweise wird Re₂O₇ eingesetzt.

Die Katalysatoren können je nach dem Herstellungsverfahren in Pulverform, in Form von Formkörpern wie Strängen, Tabletten, Pellets oder als Festbett erhalten werden.

Die aliphatische Dicarbonsäure weist mindestens 3 Kohlenstoffatome, besonders bevorzugt mindestens 4 Kohlenstoffatome auf. Die Zahl der Kohlenstoffatome bezieht sich auf die einzelne Säure und beinhaltet die Carboxylgruppen. Derivate der Carbonsäure weisen entsprechend mehr Kohlenstoffatome auf.

Dabei enthält die Carbonsäure gemäß einer Ausführungsform keine OH-Gruppen in Nachbarstellung zu den Carboxylgruppen beziehungsweise überhaupt keine Hydroxylgruppen.

Die aliphatische Dicarbonsäure weist im allgemeinen 3 bis 30, insbesondere 4 bis 20, speziell 4 bis 10 Kohlenstoffatome auf. Der aliphatische Rest kann dabei linear oder verzweigt sein. Er kann eine oder mehrere Doppel- und/oder Dreifachbindungen im Gerüst aufweisen. Übliche Anhydride oder Ester können anstelle der freien Dicarbonsäure im erfindungsgemäßen Verfahren eingesetzt werden.

Beispiele geeigneter Dicarbonsäuren sind Bernsteinsäure, Fumarsäure, Maleinsäure, und Adipinsäure.

Beispiele für Lactone sind Butyrolacton, Methylbutyrolactone oder Caprolacton.

Liegen Doppel- oder Dreifachbindungen im Gerüst vor, so werden sie bei der Hydrierung zu den gesättigten Verbindungen mithydriert. Liegen Carbonylgruppen vor, so werden auch diese hydriert.

Die erhaltenen Alkohole sind vielseitig einsetzbar, beispielsweise als Lösungsmittel, Zwischenprodukte oder Alkoholkomponenten für Polymere.

Dicarbonsäuren können bei der Hydrierung unverdünnt oder in Lösung oder Suspension eingesetzt werden. Als Lösungsmittel eignen sich alle unter den Reaktionsbedingungen inerten Stoffe, wie Wasser, Dioxan, Tetrahydrofuran, Ethylenglykolether, Kohlenwasserstoffe wie Hexan oder Alkohole wie Methanol, Ethanol oder das in der Umsetzung erhaltene Reaktionsprodukt. Bevorzugt werden Wasser und/oder bei der Reaktion entstehende Alkohole als Lösungsmittel eingesetzt. Die Hydrierung kann kontinuierlich oder diskontinuierlich durchgeführt werden. Dabei werden die Katalysatoren bei der diskontinuierlichen Fahrweise beispielsweise in Pulverform, bei der kontinuierlichen Fahrweise beispielsweise in einem Festbett angeordnet. Bei kontinuierlicher Fahrweise kann eine Produktrückführung vorgesehen werden.

Die Temperatur bei der Hydrierung beträgt vorzugsweise maximal 200°C.
Die Hydriertemperaturen liegen vorzugsweise im Bereich von 30 bis 200°C, besonders bevorzugt 100 bis 185°C, insbesondere 120 bis 170°C. Der Reaktionsdruck, der in der Regel mit Wasserstoff eingestellt wird, liegt vorzugsweise im Bereich von 1 bis 350 bar. Die Umsetzung kann in der Gasphase durchgeführt werden, wobei der Druck vorzugsweise 1 bis 80 bar beträgt. Bei der Durchführung der Reaktion in der Flüssigphase beträgt der Druck vorzugsweise 20 bis 330 bar, besonders bevorzugt 100 bis 300 bar.

Die Hydrierung kann dabei in Gegenwart von Wasser durchgeführt werden.

Bei der Hydrierung von aliphatischen Dicarbonsäuren oder Anhydriden oder Estern davon in Gegenwart des erfindungsgemäßen Katalysators können neben aliphatischen Diolen auch Lactone durch Ringschluß gebildet werden. Durch geeignete Auswahl des mindestens einen weiteren Elements des Katalysators kann dabei die Selektivität in bezug auf Diol oder Lacton gesteuert werden. Beispielsweise werden bei Verwendung von Kobaltacetat als Quelle für das weitere Element überwiegend Methylbutyrolactone bei der Reduktion von Itaconsäure gebildet. Bei Verwendung von Palladiumacetat als Quelle für das weitere Element wird überwiegend 2-Methylbutandiol gebildet.

Die Erfindung wird nachstehend anhand von Beispielen näher erläutert.

### Beispiele

### Beispiel 1

In einem Metallautoklaven wurden 0,1 g PtO₂, 0,2 g Re₂O₇, 0,1 g Silberacetat und 9 g Wasser vorgelegt. Anschließend wurde 60 bar Wasserstoff aufgepreßt und unter Rühren auf 270°C aufgeheizt. Nach 1 Stunde wurde auf Raumtemperatur abgekühlt, der Autoklav entspannt und 1 g Adipinsäure zugegeben. Danach wurden 100 bar Wasserstoff aufgepreßt und es wurde unter Rühren auf 150°C aufgeheizt. Nach 2 Stunden wurde wieder abgekühlt und entspannt. Der Reaktionsaustrag wurde gaschromatographisch analysiert. Es fanden sich bei vollständigem Adipinsäureumsatz 81,3% 1,6-Hexandiol. Der Rest bestand aus n-Hexanol, 6-Hydroxycapronsäure und dem Ester aus Hexandiol und Hydroxycapronsäure.

### Beispiel 2

Analog Beispiel 1 wurde Itaconsäure hydriert. Bei 100% Umsatz fanden sich im Reaktionsaustrag 45,2% 2-Methylbutandiol und 47,9% Methylbutyrolactone. Der Rest bestand überwiegend aus 3-Methyltetrahydrofuran, 2-Methylbutanol und 3-Methylbutanol.

### Beispiel 3

Analog Beispiel 2 wurde statt Silberacetat Kobaltacetat für die Katalysatorpräparation eingesetzt. Nach Hydrierung analog Beispiel 2 fanden sich im Reaktionsaustrag 17,1% 2-Methylbutandiol und 75,6% Methylbutyrolactone. Der Rest bestand überwiegend aus 3-Methyltetrahydrofuran, 2-Methylbutanol und 3-Methylbutanol.

### Beispiel 4

Analog Beispiel 2 wurde statt Silberacetat Triphenylphosphinogoldnitrat für die Katalysatorpräparation eingesetzt. Nach Hydrierung analog Beispiel 1 fanden sich im Reaktionsaustrag 62,5% 2-Methylbutandiol und 24,4% Methylbutyrolactone. Der Rest bestand überwiegend aus 3-Methyltetrahydrofuran, 2-Methylbutanol und 3-Methylbutanol.

### Beispiel 5

Analog Beispiel 2 wurde statt Silberacetat Palladiumacetat für die Katalysatorpräparation eingesetzt. Nach Hydrierung analog Beispiel 2 fanden sich im Reaktionsaustrag 76,7% 2-Methylbutandiol und 1,8% Methylbutyrolactone. Der Rest bestand überwiegend aus 3-Methyltetrahydrofuran, 2-Methylbutanol und 3-Methylbutanol.

### Beispiel 6

Analog Beispiel 2 wurde statt Silberacetat Molybdäntrioxid für die Katalysatorpräparation eingesetzt. Nach Hydrierung analog Beispiel 2 fanden sich im Reaktionsaustrag 73,3% 2-Methylbutandiol und 8,8% Methylbutyrolactone. Der Rest bestand überwiegend aus 3-Methyltetrahydrofuran, 2-Methylbutanol und 3-Methylbutanol.

### Beispiel 7

Analog Beispiel 1 wurde Maleinsäure bei 140°C hydriert. Bei 100% Umsatz fanden sich im Austrag 70% 1,4-Butandiol. Der Rest bestand überwiegend aus Tetrahydrofuran, gamma-Butyrolacton, 4-Hydroxybutyraldehyd und Butanol.

## Patentansprüche

1. Verfahren zur Herstellung von aliphatischen Diolen oder Lactonen durch Hydrierung von aliphatischen Dicarbonsäuren mit mindestens 3 Kohlenstoffatomen oder Anhydriden oder Estern davon in Gegenwart eines Pt und Re jeweils in metallischer oder oxidischer Form enthaltenden Katalysators, **dadurch gekennzeichnet, daß** der Katalysator ferner mindestens ein weiteres Element aus den Gruppen 5 bis 12 und 14 und der Lanthaniden des Periodensystems der Elemente, ausgenommen Palladium, in metallischer oder oxidischer Form enthält.

2. Verfahren nach Anspruch 1, **dadurch gekennzeichnet, daß** der Katalysator durch Reduktion einer wäßrigen Aufschlämmung und/oder Lösung von Oxiden, Oxidhydraten, Carbonaten, Nitraten, Carboxylaten, Chelaten, Sulfaten, Phosphaten und/oder Halogeniden von Pt, Re und mindestens einem weiteren Element aus den Gruppen 5 bis 12 und 14 und der Lanthaniden des Periodensystems der Elemente hergestellt wird.

3. Verfahren nach Anspruch 1 oder 2, **dadurch gekennzeichnet, daß** der Katalysator als mindestens ein weiteres Element mindestens ein Element aus den Gruppen 6, 10 und 11 des Periodensystems der Elemente in metallischer oder oxidischer Form enthält.

4. Verfahren nach einem der Ansprüche 1 bis 3, **dadurch gekennzeichnet, daß** der Katalysator Mo, Ag und/oder Au jeweils in metallischer oder oxidischer Form enthält.

5. Verfahren nach einem der Ansprüche 1 bis 4, **dadurch gekennzeichnet, daß** die Temperatur bei der Hydrierung maximal 200°C beträgt.

6. Verfahren nach einem der Ansprüche 1 bis 5, **dadurch gekennzeichnet, daß** die Hydrierung in Gegenwart von Wasser durchgeführt wird.

7. Verfahren nach einem der Ansprüche 1 bis 6, **dadurch gekennzeichnet, daß** die Pt-Komponente vor einer Reduktion mit Wasserstoff als PtO₂ vorliegt.

8. Verwendung von Katalysatoren, wie sie in einem der Ansprüche 1 bis 4 beschrieben sind, zur Hydrierung von aliphatischen Dicarbonsäuren oder Anhydriden oder Estern davon.

## Claims

1. A process for preparing aliphatic diols or lactones by hydrogenating aliphatic dicarboxylic acids having at least 3 carbon atoms or anhydrides or esters thereof in the presence of a catalyst comprising Pt and Re, in the form of the metal or an oxide in each case, wherein the catalyst further comprises at least one further element from groups 5 to 12 and 14 and the lanthanides of the Periodic Table of the Elements, with the exception of palladium, in the form of the metal or an oxide.

2. A process as claimed in claim 1, wherein the catalyst is prepared by reducing an aqueous suspension and/or solution of oxides, oxide hydrates, carbonates, nitrates, carboxylates, chelates, sulfates, phosphates and/or halides of Pt, Re and at least one further element from groups 5 to 12 and 14 and the lanthanides of the Periodic Table of the Elements.

3. A process as claimed in claim 1 or 2, wherein the at least one further element is at least one element from groups 6, 10 and 11 of the Periodic Table of the Elements in the form of the metal or an oxide.

4. A process as claimed in any of claims 1 to 3, wherein the catalyst comprises Mo, Ag and/or Au in the form of the metal or an oxide in each case.

5. A process as claimed in any of claims 1 to 4, wherein the hydrogenation temperature is at most 200°C.

6. A process as claimed in any of claims 1 to 5, wherein the hydrogenation is carried out in the presence of water.

7. A process as claimed in any of claims 1 to 6, wherein the Pt component is present in the form of PtO₂ prior to reduction with hydrogen.

8. The use of catalysts as described in any of claims 1 to 4 for the hydrogenation of aliphatic dicarboxylic acids or anhydrides or esters thereof.

## Revendications

1. Procédé pour fabriquer des diols ou des lactones aliphatiques par hydrogénation d'acides dicarboxyliques aliphatiques comportant au moins 3 atomes de carbone ou de leurs anhydrides ou esters en présence d'un catalyseur comprenant du Pt et du Re soit sous forme métallique, soit sous forme d'un oxyde, **caractérisé en ce que** le catalyseur comprend en outre au moins un autre élément provenant des groupes 5 à 12 et 14 et du groupe des lanthanides de la Classification Périodique des Eléments, excepté le Palladium, sous forme métallique ou sous forme d'un oxyde.

2. Procédé selon la revendication 1, **caractérisé en ce que** l'on produit le catalyseur par réduction d'une suspension et/ou d'une solution aqueuse constituée d'oxydes, d'hydrates d'oxydes, de carbonates, de nitrates, de carboxylates, de chélates, de sulfates, de phosphates et/ou d'halogénures de Pt, Re et d'au moins un autre élément provenant des groupes 5 à 12 et 14 et du groupe des lanthanides de la Classification Périodique des Eléments.

3. Procédé selon la revendication 1 ou 2, **caractérisé en ce que** le catalyseur contient en tant que le au moins un autre élément au moins un élément des groupes 6, 10 et 11 de la Classification Périodique des Éléments sous forme métallique ou sous forme d'un oxyde.

4. Procédé selon l'une quelconque des revendications 1 à 3, **caractérisé en ce que** le catalyseur contient du Mo, de l'Ag et/ou de l'Au soit sous forme métallique, soit sous forme d'un oxyde.

5. Procédé selon l'une quelconque des revendications 1 à 4, **caractérisé en ce que** la température d'hydrogénation s'élève au maximum à 200°C.

6. Procédé selon l'une quelconque des revendications 1 à 5, **caractérisé en ce que** l'on effectue l'hydrogénation en présence d'eau.

7. Procédé selon l'une quelconque des revendications 1 à 6, **caractérisé en ce que** le composant de Pt se présente sous forme de PtO₂ avant une réduction avec de l'hydrogène.

8. Utilisation des catalyseurs tels que décrits dans l'une quelconque des revendications 1 à 4, pour hydrogéner des acides dicarboxyliques aliphatiques ou leurs anhydrides ou esters.
